# EUROPEAN PATENT SPECIFICATION

(11) **EP 2 925 330 B1**
(45) Date of publication and mention of the grant of the patent: **21.04.2021**
(21) Application number: 13805600.7
(22) Date of filing: 02.12.2013
(51) Int. Cl.: A61K 31/717, A61P 3/04, A61P 3/06, A61P 3/08, A61P 3/10

(54) **PHARMACEUTICAL COMPOSITIONS FOR TREATING OBESITY**
PHARMAZEUTISCHE ZUSAMMENSETZUNGEN ZUR BEHANDLUNG VON ADIPOSITAS
COMPOSITIONS PHARMACEUTIQUES POUR LE TRAITEMENT DE L'OBÉSITÉ

(30) Priority: 03.12.2012 IT FI20120268
(43) Date of publication of application: 07.10.2015
(73) Proprietor: Diopeite SA, 6900 Lugano (CH)
(72) Inventor: BIZZINI, Bernard, 11000 Carcassonne (FR)
(74) Representative: Sarpi, Maurizio
(86) International application number: PCT/IB2013/060558
(87) International publication number: WO 2014/087317

(56) References cited:
- EP-A1- 0 503 496
- WO-A1-03/103640
- WO-A2-2007/036920
- AU-B2- 657 301
- DE-A1- 10 339 354
- US-A1- 2010 291 238
- US-A1- 2012 122 691
- KOJIMA S ET AL: "Hydrazine-conjugated cellulose for adsorption of glycated proteins.", THERAPEUTIC APHERESIS : OFFICIAL JOURNAL OF THE INTERNATIONAL SOCIETY FOR APHERESIS AND THE JAPANESE SOCIETY FOR APHERESIS MAY 1999, vol. 3, no. 2, May 1999 (1999-05), pages 186-189, XP055072175, ISSN: 1091-6660
- KOBAYASHI AKIRA ET AL: "In vitro evaluation of dextran sulfate cellulose beads for whole blood infusion low-density lipoprotein-hemoperfusion.", THERAPEUTIC APHERESIS : OFFICIAL JOURNAL OF THE INTERNATIONAL SOCIETY FOR APHERESIS AND THE JAPANESE SOCIETY FOR APHERESIS OCT 2002, vol. 6, no. 5, October 2002 (2002-10), pages 365-371, XP002706452, ISSN: 1091-6660

## Description

### Field of the invention

The present invention relates to the field of pharmaceutical or dietary products for use in the treatment of obesity and of overweight conditions; more specifically, the invention relates to compositions based on activated cellulose having a sequestering action and/or a competitive action against the intestinal absorption of fat and sugar foods.

### State of the art

To date there are no dietary-slimming products that are able to perform their function in an optimal way.

All times any exogenous event, such as diet, fast or a pharmacological treatment, causes a physiological-functional deficiency of lipid and/or carbohydrate material in the body, this triggers mechanisms for greater use of the deposited organic material, the subsequent biosynthesis of endogenous material and, due to a feedback effect, it activates the request of exogenous nutritional material, or in other words it has the effect of stimulating appetite.

This stimulation occurs through the control of the Central Nervous System (CNS) and develops through stimulation of bio-mechanisms that regulate the functions of appetite and of the nutrients assimilation. To the promotion regulated by the CNS also aspects of psychological nature contribute such as, for instance, the sense of deprivation and the reaction to forced abstinence that result in the consequent desire to access to food, in particular to the "forbidden" food. L. Montgomery et al. in Biochemistry: medical aspects of biological, 1975, Hermes Ed., have shown that a sudden drop in calories intake generates a subjective and objective state of stress resulting from:
- feeling of food deprivation (subjective stress) that for the rebound effect stimulates the appetite and the share of assimilation of available nutrients;
- increased Metabolism of lipids in the adipocyte deposit which, due to a feedback effect, promotes in its turn the appetite stimulus and the gastrointestinal assimilation of nutrients (objective stress);
- subjective-type stress also suffered because of the diversity in the type of diet imposed: certain organoleptic sensory habits are completely eliminated and replaced with tastes and flavours of different palatability.

The containment of the stress state is very important not only for achieving the result, but also and above all for its preservation, given the risk of relapse. In this context, the "logical" function of a dietary-slimming product is to assist the results on the control/reduction of body weight obtainable with a proper diet, which is able not to induce a state of stress, more or less associated with an increased physical activity.

This adjuvant effect, in order to allow the removal of stress conditions, must primarily enable to:
a) minimize the jump of caloric intake needed to restore the proper body weight;
b) have access to all types of food and eliminate possible deficiencies of essential elements;
c) include in the diets foods of the desired palatability;
d) be able to maintain the results obtained with the diet, without detriment for the subject.

To achieve the goal of having available a dietary-slimming product that meets the above said requirements, we have considered the following two additional aspects:
a. the physiological mechanism of absorption of the two types of nutrients, lipids and carbohydrates, which are the compounds directly involved in metabolic dysfunctions that cause numerous pathophysiological alterations, including obesity;
b. the type of activity performed by a series of dietary products among the most used nowadays, such as the dietary fibres and dextran polymers.

The absorption of nutrients requires an enzymatic activity that, during the complex digestive process, has the function to reduce macromolecular foods to the mono-structural chemical status; only the lipids are not totally catabolized to the unit and they may be absorbed even as fragments. Their absorption occurs according to two possible mechanisms, passive and of active transport, and it involves the chemical, physico-chemical and structural properties of the fats themselves.

The lipids, for their absorption, are emulsified at the level of the intestinal lumen, digested by enzymes and absorbed by the cells of the intestinal mucosa; the passage of the mixed micelles to the mucosa is a passive process which takes place by diffusion.

This process essentially concerns the triglycerides containing long chain fatty acids, which constitute the majority of the lipids ingested with food, the phosphoglycerides, the cholesterol and its esters. The digestion of triglycerides stops almost completely to the 2-monoglyceride; these and the fatty acids derived from the hydrolysis, may pass through the cellular membranes and thus be absorbed, by diffusion, within the cells which form the ileum and jejunum mucosa.

The amount of lipids that can be stored in adipocytes seems to be unlimited, against an organic requirement that is practically zero for the body's ability to endo-synthesize saturated, monoenoic and polyenoic, fatty acids; the only fatty acids that are essential for the organism, to be provided with the diet, are in fact the essential fatty acids of the type of linoleic acid, essential for maintaining the functional state of the cellular membranes, for the regulation of oxidative phosphorylation and for the biosynthesis of prostaglandins/prostacyclines.

Carbohydrates have an essentially dynamic function, i.e. they are used by the body as a source of energy. It follows, for example, that while the amount of protein to be introduced with the diet should be fairly regular in time, the amount of sugars, such as on the other hand the amount of fats, must vary in relation to the working conditions performed by the organism.

Some sugars, particularly pentose sugars, pass the intestinal barrier by simple passive diffusion; other sugars, in particular D-glucose, D-fructose and, probably, mannose are actively transported also against a concentration gradient. This means that these sugars are absorbable by the intestine even if their concentration in the blood is much higher.

Under physiological conditions, however, the concentration of sugars is always greater in the intestine than in the blood; it is likely, therefore, that the mechanism of absorption against gradient be useful to avoid the passive diffusion of the same in the opposite direction, i.e. from blood to intestinal lumen, which could occur, for instance, during a fast or a very strict diet.

The splitting of disaccharides and the absorption of monosaccharides mainly occurs in the microvilli located in the first portion of the jejunum; the two processes promote each other so that a few of monosaccharides produced by hydrolysis goes back in the intestinal lumen.

The process of digestion and absorption of sugars contained in foods occurs gradually; it is proved that the rate of absorption by the intestinal lumen is fairly constant and that occurs, within rather wide limits, irrespective of their total amount or concentration with which they are introduced in the intestine.

A typical example of nutraceutical treatment, which should act at the intestinal level on the absorption of fats and sugars, is represented by dietary fibres and by dextran polymers.

The fibre-rich diets are generally used as a prophylactic and therapeutic means in the functional regulation of intestinal constipation. The dietary fibres can act as laxatives with various mechanisms: they can, for instance, bind water and ions in the intestinal lumen; it is then assumed that they also perform a dietetic effect.

Some components of the dietary fibres, such as pectin, can be digested by bacteria in the colon thus creating metabolites that contribute to the laxative effect by acting on the osmotic activity.

The fibres may also support the growth of intestinal bacteria by increasing the faecal mass; it is also possible that the bacterial fermentation produced by the fibres generates metabolites affecting the fluid mechanism and the transport of electrolytes. The fibres in a diet, ultimately, interact with the process of constipation by increasing the faecal mass, its water content and the time of colonic transit. Their alleged effectiveness as appetite suppressant has not yet been well clarified and demonstrated.

Cellulose and dextran seem to be able to bind and reduce the intestinal absorption of many substances, such as cardio-active glycosides, salicylates, and nitrofurantoin.

Among examples known in the art relating to compositions based on differently mixed or derivatised celluloses, the following are cited:
Patent Documentsa. US Patent No. 3, 023, 104 -crystalline cellulose aggregates administered in association with low-calorie food mixtures;
b. Japanese Patent No. 80165532 -substances inhibiting the absorption of glucose, such as phloretin, linked to cellulose and dextrans;
c. European Patent No. 502 666 -water-soluble cellulose ethers having high density suitable for selectively reducing the levels of cholesterol and of low density lipoproteins;
d. International patent application No. WO 96/14851 -cationic cellulose derivatives linked to quaternary ammonium groups useful for reducing serum cholesterol levels. EP 0503496 A1 discloses the use of water soluble cellulose selected from methylcellulose, hydroxypropyl cellulose, methylethyl cellulose, hydroxyethyl cellulose and hydroxyl propylmethyl cellulose, for reducing total plasma cholesterol levels.

US2010/291238 A1 discloses that ethanol treated water soluble cellulose derivative methocel is useful to treat obesity.

DE 10339354 A1 discloses the subject matter of claims 1, 6-8 (i.e. a composition for treating metabolic syndrome, comprising swellable or gel-forming satiating and weight reduction agent (such as carboxymethylcellulose) and hypocholesterlomic and (or antidiabetic agents for use for treating metabolic syndrome.

AU657301 B2 discloses foodstuff and compositions for reducing total plasma cholesterol by using water soluble cellulose ethers especially hydroxypropilmthylcellulose

WO 2007/036920 A2 discloses that hydrolysed oxised cellulose is used as anti-hyperlipidemic and serum glucose regulation. The compounds could be integrated into foods.

WO 03/103640 A1 discloses cellulose as cholesterol lowering agent.

### Not Patent Documents

Kojima et al., discloses hydrazine-conjugated cellulose for adsorption of glycated proteins for preventing advanced glycation damage in diabetic patients.

Among the synthetic derivatives having a similar activity the diethylaminoethyl-dextran as sequestering agent of fatty acids may be cited as an example.

According to the state of the art, other products are used with the purpose of giving a feeling of satiety such as barley extracts or other vegetal products more or less supported in maltose-type materials, semi-synthetic celluloses, gums, polyacrylic resins and hydrophilic derivatives thereof. However these products are used as "alternative meals" or anyway, in view of their limited effectiveness, they require a simultaneous significant reduction in the diet. This makes this type of treatment subject to the same side effects of the fast and of the strict, generic diets, because:
- the processes initiated at the level of intestinal lumen activate the endogenous biochemical processes assigned to demand for nutrients;
- the sense of satiety is only temporary, and affects the psychological aspects related to the state of deprivation, with consequent activation of feedback mechanisms;
- the state of stress resulting from a different palatability of the food remains unchanged.

In view of what reported above, it is therefore evident how it still unsolved the problem of having available a dietary-slimming product that is really effective but also capable of minimizing the stress state associated to the drastic reduction in calories intake and to the change in the type of food during diets.

Despite the prior art none of the technical documents concerns a pharmaceutical or dietary composition suitable for oral administration in the treatment of obese or overweight subjects and of subjects suffering from hyperglycaemia and/or from hyperlipidaemia, according to claim 1, comprising as active principle activated cellulose obtained by hot alkaline treatment of granular cellulose, followed by cooling and pH neutralization,, possibly derivatised to form the group consisting of cellulose-oleylamine, cellulose-glucosamine and cellulose-oleylamine-glucosamine, alone or in combination with further active principles.

### Summary of the invention

The Applicant has now surprisingly found that the granular cellulose activated by an alkaline heat treatment is able to trap fats and alimentary sugars up to three to four times its own weight and they are kept entrapped in the extent of about 80%, and are not released by subsequent washings with suitable solvents or suitable buffers.

The cellulose thus activated in fact preserves the properties of indigestibility and not absorbability of the non-activated cellulose, also being able at the same time to also promote the entrapment of alimentary fats and sugars so as to render them non-absorbable. If appropriately derivatised, such activated cellulose is further able to compete at absorption sites with the alimentary fats and sugars themselves.

The cellulose thus activated in fact preserves the properties cellulose not being activated, however, at the same time also able to promote the entrapment of fat and sugar in foods so as to make them non-absorbable. If suitably derivatised, such activated cellulose is also able to compete at the level of absorption sites with the same fat and sugar food.

Thanks to these characteristics, the activated cellulose of the invention may be used in pharmaceutical or dietary compositions having slimming action.

Scope of the present invention is therefore a pharmaceutical or dietary composition suitable for oral administration in the treatment of obese or overweight subjects and of subjects suffering from hyperglycaemia and/or from hyperlipidaemia, comprising as active principle activated cellulose obtained by hot alkaline treatment of granular cellulose, followed by cooling and pH neutralization, , possibly derivatised to form the group consisting of cellulose-oleylamine, cellulose-glucosamine and cellulose-oleylamine-glucosamine, alone or in combination with further active principles, as stated in the independent claim 1.

A further subject of the present invention is the use of activated cellulose, possibly derivatised, for the manufacture of a medicament for the treatment of obese or overweight subjects, and of subjects suffering from hyperglycaemia and/or hyperlipidaemia.

Further characteristics and advantages of the invention will be illustrated in the following detailed description.

### Detailed description of the invention

The compositions of the present invention have shown to be able to allow a correct treatment of the body overweight, of obesity, of hyperglycaemia and hyperlipidaemia, thus minimizing the state of stress linked to a drastic reduction of the calories intake and to a change in the type of foods included in the diet.

In particular, the present compositions are capable to inhibit the gastrointestinal absorption of alimentary fats and sugars by acting according to two different mechanisms of action:
a) structural entrapment of alimentary fats and sugars that, in this way, are no more bioavailable for absorption;
b) fixation on the sites of the gastrointestinal membrane that, so occupied, does not allow the passage of alimentary fats and sugars.

The cellulose activated according to the process described in the following acts in line with the first mechanism a) in order to, when ingested during meals, sequester alimentary fats and sugars that in this way are no longer absorbable; the sequestering power is strong and fat and sugars are not released even after washing with suitable solvents for fats, or with suitable buffers for sugars.

The derivatives of cellulose activated with oleylamine and/or with glucosamine, that are the conjugates cellulose-oleylamine, cellulose-glucosamine and cellulose-oleylamine-glucosamine, act in accordance with the competitive mechanism b) respectively in respect of the absorption of fats or sugars.

In particular, the complex conjugate cellulose-oleylamine-glucosamine is able to occupy the sites of absorption of fats and sugars, blocking their functionality due to the indigestibility and not absorbability of the cellulosic carrier substrate.

The synergistic effect against the absorption of fats and sugars results in a significant decrease of the body weight.

In the association with the activated cellulose, the alternative use of the conjugates cellulose-oleylamine or cellulose-glucosamine is found useful in the treatment, respectively, of hyperlipidaemia or of hyperglycaemia.

The significant extent of the effect requires the integration of the treatment in particular as regards the liposoluble vitamins and the unsaturated, short-chain, fatty acids of the type of linoleic acid.

The radicals bound to cellulose fixate on the absorption sites of the enteric membrane and remain therein because of indigestibility and not absorbability of the cellulose.

The present compounds dietary-slimming compounds having sequestering action (i.e. the present activated cellulose) or having a competitive action (i.e. the present derivatives of activated cellulose, that are the conjugates cellulose-oleylamine, cellulose-glucosamine and the complex conjugate cellulose-oleylamine-glucosamine) may be used alone or in combination between each other depending on the type and severity of the disease concerned.

The preferred mode of administration of the present compositions is during meals, when they constitute a real barrier to the absorption of alimentary fats and sugars.

The preferred administration forms of the present compositions are all forms suitable for oral administration, and are typically the granular form or the form incorporated into bread, crackers or other suitable foods.

The dietary-slimming treatment, antilipemic and/or antiglycaemic, carried out by the administration of the present compositions, requires no change in the type of food or significant lowering of the calories.

The treatment, due to the high level of effectiveness, may instead require daily supplementation with liposoluble vitamins and linoleic acid.

The use in combination of the products of the invention, having sequestering action of fats and sugars and competitive action towards their absorption, allows to obtain a significant reduction of body weight, and/or of hyperglycaemia and/or of hyperlipidaemia in a short time, even in the presence of a normal, not low-calorie, diet, and eating the favourite food for the subject, thus eliminating the conditions typical of traditional diets that are source of stress.

The content of activated cellulose and of derivatives thereof in the present compositions is determined so as to allow the best effectiveness. Pharmaceutically acceptable excipients, diluents and adjuvants, commonly used in dietary or pharmaceutical compositions for oral use, may be comprised in the present compositions.

By the term "activated cellulose" according to the present invention is meant the product obtained by alkaline heat treatment of granular cellulose; this process is preferably carried out by heat treatment of granular cellulose with aqueous solutions of a strong base, such as sodium hydroxide, for example at a temperature of about 90°C. By cooling with ice a suspension is obtained that, properly neutralized in pH, washed and dried, allows yielding the desired product.

The activated cellulose thus obtained may then be derivatised, in particular with oleylamine and/or glucosamine, with known procedures and under conditions known to any expert in the field.

Hereinafter, the present invention will be described in more detail through examples . The following examples are for illustrative purposes only and it will be apparent to those of ordinary skill in the related art that the scope of this invention is not limited by the examples but defined by the appended claims.

### Chemical experimental part

### Example 1 -Activation of the cellulose (not object of the present invention)

In a reactor, under stirring, 20 litres were poured of an aqueous solution of sodium hydroxide 2 N, which were then brought to a temperature of 70°C. It is then added, still under vigorous stirring, 1 kg of granular cellulose and the suspension was brought to 90°C. It is left to react for 10 minutes then rapidly cooling by addition of ice.

It is filtered and the filtrate was first washed with acetate buffer at pH 5.0 up to pH neutralization, subsequently washed with water and dried in a stove overnight at 65°C. The yield of activated cellulose was 95%. The final pH of a 10% suspension of the obtained product was between 6.5 and 7.0.

### Example 2 -Synthesis of the derivate activated cellulose-oleylamine (not object of the present invention)

200 g of activated cellulose obtained as described in Example 1 were oxidized with 3 litres of sodium monoiodoacetate (MIA) 0.1 M, protected from light and under stirring, for 6 hours at room temperature. The so oxidized cellulose was collected in a Buchner, washed with water then with carbonate buffer 0.1 M at pH 8.8 and then collected.

To a solution of 14 g of oleylamine in 250 ml of ethanol 250 ml of carbonate buffer were added in order to maintain the oleylamine in solution. To this solution 150 g of humid oxidized cellulose were added (corresponding to 50 g of dry cellulose) and the conjugation was allowed to take place, protected from light and under stirring overnight at room temperature.

When conjugation has occurred, 1 g of sodium borohydride was added and allowed to react for 10 minutes. The conjugate was collected in a Buchner, washed with water, and dried over P205 under vacuum.

### Example 3 -Synthesis of the derivate activated cellulose-glucosamine (not object of the present invention)

200 g of activated cellulose obtained as described in Example 1 were subjected to oxidation with 3 litres of sodium monoiodoacetate (MIA) 0.1 M, protected from light under stirring for 6 hours at room temperature. The so oxidized cellulose was collected on Buchner, washed with water then with 0.1 M carbonate buffer at pH 8.8.

To a solution of 11 g of glucosamine HCI dissolved in water and diluted to 500 ml with carbonate buffer 0.1 M at pH 8.8, 150 g of humid oxidized cellulose were added (corresponding to 50g of dry cellulose) and the conjugation was allowed to take place, protected from light, under stirring overnight at a temperature of 4°C and, subsequently, for 4 hours at room temperature.

1 g of sodium borohydride was added, allowing then to react for 10 minutes. The conjugate was collected on a Buchner, washed with water, and then dried under vacuum over P205.

### Example 4 -Synthesis of the complex conjugate activated cellulose-oleylamineglucosamine (not object of the present invention)

200 g of activated cellulose prepared as described in Example 1 were subjected to oxidation with 3 litres of sodium monoiodoacetate (MIA) 0.1 M, protected from light under stirring for 6 hours at room temperature.

The so oxidized cellulose was collected on Buchner, washed with water then with carbonate buffer 0.1 M at pH 8.8. To a solution of 7 g of oleylamine in 200 ml of ethanol 200 ml were added of a solution of 5.5 g of glucosamine in carbonate buffer 0.1 M at pH 8.8.

To this mixture 150 g of humid oxidized cellulose were added (50 g of oxidized dried cellulose) and the conjugation was allowed to take place, protected from light, under stirring, overnight at room temperature.

At the end of conjugation 1 g of sodium borohydride was added, which was allowed to react for 10 minutes. At the end, the conjugate was collected on Buckner, washed with water, and then dried under vacuum over P20 5.

### Pharmacological experimental part

### Example 5 -Evaluation of the sequestering power of fats and sugars of the activated cellulose (not object of the present invention)

In a chromatographic column with a diameter of 1 cm, it is deposited 1.0 g of granular activated cellulose obtained as described in Example 1, corresponding to a chromatographic bed height of approximately 4 cm.

4.5 ml of oil (4.06 g) were added, or 10.0 ml of a 40% glucose solution, and allowed to elute. When elution has occurred, the cellulose matrices with fats or sugars incorporated were weighed.

Subsequently 20 ml of acetone are eluted through the columns with oil incorporated, and 50 ml of PBS buffer at pH 7.4 in the columns with sugar incorporated. Then the cellulose matrices with fats or sugars still incorporated after washing with solvents, were weighted obtaining the following results:
- incorporated oil: from 2.5 to 3.5 times the weight of the granular activated cellulose; on average 3 times the weight of the same;
- oil still incorporated after washing with acetone: 80-85% of total incorporated;
- incorporated glucose: from 2.5 to 3.0 times the weight of the granular activated cellulose; on average 2.8 times the weight of the same; -glucose still incorporated after washing with PBS: 75 -80 % of the total incorporated.

### Example 6 -Analysis in rats of the competition in fats absorption of the celluloseoleylamine derivative

Adult Wistar rats weighing 200 g, fasted for 24 hours, were fed by means of a probe with 1.0 g/kg of cellulose-oleylamine prepared as described in Example 2 in aqueous suspension. After 15 minutes, to these animals, always by means of a gastric probe, 5.0 ml/kg of oil of sunflower seeds were administered.

In parallel, a group of animals was treated only with sunflower seeds oil (controls) . After 2, 4 and 8 hours from the treatment, 1 ml of blood is taken from the animals by open sky intra-cardiac injection and, in these samples, the total lipid content was determined. The results obtained are the following:
- percentage increase in blood levels of total lipids in control animals: 47% at 4 hours;
- percentage increase in blood levels of total lipids in the treated animals: 6% at 2 hours.

### Example 7 -Analysis in rats of the competition in sugars absorption of the cellulose-glucosamine derivative

Adult Wistar rats weighing 200 g, fasted for 24 hours, were fed by a probe with 1.0 g/kg of cellulose-glucosamine prepared as described in Example 3 in aqueous suspension. After 15 minutes, to these animals, always by means of a gastric probe, 10 ml/kg of a 40% solution of glucose were administered.

In parallel, a group of animals was treated only with the glucose solution (controls) . After 2, 4 and 8 hours from the treatment, 1 ml of blood was taken from the animals by open-sky intra-cardiac injection and the content of total glucose was determined on these samples. The results obtained were the following:
- percentage increase in blood levels of glucose in control animals: 32% at 2 hours;
- percentage increase in blood levels of glucose in the treated animals: 9% at 2 hours.

### Example 8 -Analysis of the use of complex conjugate cellulose-oleylamineglucosamine on the ponderal growth curves of rats

Wistar rats in accretion stage, weighing 80 g, were subjected to treatment with the conjugate cellulose-oleylamine-glucosamine prepared as described in Example 4, at a dose of 100 mg/kg/day, associated with feed enriched in fats and sugars by addition of seeds oil and glucose.

The treatment was continued for 30 days checking periodically, every 5 days, the trend of body weight. At the beginning (time zero) and at the end of treatment blood levels of glucose and of the total lipids were determined. In parallel, a group of animals was treated only with the diet enriched in fats and sugars.

The results of this experiment were as follows. The body weight of the animals treated with the conjugate, detected at the end of treatment, was lower than the control animals in the order of 23%. The blood levels of sugars and lipids, at the end of treatment, were in the normal range for the treated animals, while in the controls the blood levels were increased with respect to the normal values, of 35% for sugar levels and of 28% for fats levels.

### Example 9 -Analysis of the use of the association of sequestering granular cellulose and of cellulose-oleylamine-glucosamine on the trend in weight of adult rats

Adult Wistar rats, weighing 250 g, in a normal diet, were subjected to treatment with the association of granular activated cellulose obtained as described in Example 1, at a dose of 50 mg/kg, and the conjugate cellulose-oleylamine-glucosamine prepared as described in Example 4, at a dose of 50 mg/g, for a period of 30 days.

In parallel, a group of animals received only the normal feed (controls) . At the beginning and at the end of treatment the value of body weight was determined.

The results obtained in this experiment were the following. The body weight of the control animals was maintained in the same order for the whole period of the test, the body weight of the treated animals was reduced by 22%.

### Clinical experimental part

### Example 10 -Use of cellulose-glucosamine in the control of hyperglycaemia in a diabetic subject

Volunteer subjects, males and females, with ascertained diagnosis of diabetes and under insulin treatment, were treated for 60 days with the conjugate celluloseglucosamine prepared as described above in Example 3, at a dose of 5 g twice a day during the main meals, at the same time receiving insulin treatment as per medical prescription. At various times of the day, and typically after 3 hours from the treatment, the blood levels of glucose were checked by finger prick testing.

The deviations of glucose levels were checked with respect to the maximum limit of normal values. As controls the same patients were taken, but kept under control for 60 days before commencement of the trial.

It was thus observed that, before treatment, the deviation of the glucose rates compared to the maximum normal limit could undergo variations comprised averagely between 30 and 80% depending on the type of food taken by the patient; while during treatment and maintaining the same type of feeding, the deviation did never exceed the limit of 10%.

### Example 11 -Use of cellulose-oleylamine in the control of hyperlipidaemia in subjects suffering from dyslipidaemia

Volunteer subjects suffering from hypercholesterolemia were subjected to experiments in cross-over rates in order to verify the influence on cholesterol blood levels of the treatment with the cellulose-oleylamine derivative prepared as described above in Example 2.

The patients were kept under examination for 60 days, controlling the type of feeding assumed and determining, periodically every 10 days, the blood rates of cholesterol. After this period they were subjected to treatment with the conjugate cellulose-oleylamine at a dose of 5 g twice daily taken during meals. The treatment was continued for 60 days, checking periodically, every 10 days, the blood rates of cholesterol.

During the control stage, before the treatment, it was observed that the rates of hypercholesterolemia were maintained constant throughout that period. Instead, as a consequence of the treatment, blood levels of cholesterol was significantly lowered in all patients, reaching at the end of the treatment a value in the normal range in 60% of the same patients.

### Example 12 -Use of the association of activated cellulose and celluloseoleylamine-glucosamine in the reduction of body weight in obese subjects

Obese volunteers subjects, with a surplus of weight with respect to the normal of more than 20 kg, have been subjected, for a period of 6 months, to a treatment with the association of activated cellulose obtained as described in Example 1 at a dose of 2.5 g and of the conjugate cellulose-oleylamine-glucosamine as in Example 4 at a dose of 2.5 g.

The association was given twice/day during meals. In the morning, subjects also received a supplement tablet of linoleic acid and liposoluble vitamins, in order to compensate for the corresponding products sequestered and not absorbed.

Periodically, every 7 days, body weight was checked and throughout the period of the test subjects received the normal diet which they were accustomed.

The weight loss due to the treatment was in the order of 3 -3.5 kg/month. It was also verified that the treatment did not give rise to side effects and was very well tolerated.

### Example 13 -Use of activated granular cellulose or of cellulose-oleylamineglucosamine in the control of body weight in overweight subjects

Overweight volunteer subjects, having a surplus of weight compared to the normal of less than 20 kg, have been subjected, for a period of 6 months, to the 5 following treatment:
1 st group: activated cellulose of Example 1 at a dose of 5.0 g twice in a day during meals;
2nd group: cellulose-oleylamine-glucosamine conjugate of Example 4 at a dose of 5.0 g twice in a day during meals.

In the morning, the subjects received a supplement tablet based on linoleic acid and liposoluble vitamins to compensate for the corresponding substances nonabsorbed.

Throughout the period of the test, the subjects received the normal feeding that they were used to and periodically, every 7 days, their body weight was checked. It was observed that the loss of weight due to the treatment with sequestering cellulose (activated) was in the order of 2.5 -3.0 kg/month, and in the same order (2.7 kg/month) was the result obtained in the treatment with the conjugate cellulose-oleylamineglucosamine. The treatments also did not give rise to side effects, and were perfectly tolerated.

The present invention is described herein making reference to a preferred embodiment.

## Claims

1. A pharmaceutical or dietary composition suitable for oral administration in the treatment of obese or overweight subjects suffering from hyperglycaemia and/or from hyperlipidaemia, comprising as active principle activated cellulose obtained by hot alkaline treatment of granular cellulose, followed by cooling and pH neutralization, possibly derivatised to form a conjugate selected from the group consisting of cellulose-oleylamine-glucosamine, alone or in combination with further active principles.

2. The composition according to claim 1, wherein said treatment of granular cellulose is carried out at temperature of approx. 90°C with an aqueous solution of a suitable strong base, preferably sodium hydroxide.

3. The composition according to claim 1, comprising as active principle activated cellulose derivatised to form a conjugate selected from the group consisting of celluloseoleylamine, cellulose-glucosamine and cellulose-oleylamine-glucosamine.

4. The composition according to claim 1, comprising activated cellulose in combination with at least a conjugate thereof as defined in claim 1.

5. The composition according to any of the previous claims, in granular form, possibly incorporated in foodstuff, such as cookies, crackers, bread sticks and similar.

6. The composition according to any of the previous claims, further comprising one or more pharmaceutically acceptable adjuvants and/or excipients suitable for oral administration of the composition.

7. Activated cellulose obtained by hot alkaline treatment of granular cellulose, followed by cooling and pH neutralization, possibly derivatised to form a conjugate selected from the group consisting of cellulose-oleylamine, celluloseglucosamine and cellulose-oleylamine-glucosamine, alone or in combination with further active principles, for use in the treatment of obesity or overweight of obese or overweight subjects, and for use in the treatment of hyperglycaemia and/or hyperlipidemia in subjects suffering from hyperglycaemia and/or hyperlipidemia.

8. Activated cellulose for use according to claims 7, wherein said activated cellulose is derivatised to form a conjugate selected from the group consisting of cellulose-oleylamine, cellulose-glucosamine and cellulose-oleylamine-glucosamine.

9. Activated cellulose for use according to claims 7 and 8, wherein said activated cellulose, its derivative cellulose-oleylamine-glucosamine, or combinations thereof, are used in the treatment of obese or overweight subjects.

10. Activated cellulose for use according to claims 7 and 8, wherein said derivative cellulose-oleylamine, alone or in combination with activated cellulose, is used in the treatment of subjects suffering from hyperlipidaemia.

11. Activated cellulose for use according to claims 7 and 8, wherein said derivative cellulose-glucosamine, alone or in combination with activated cellulose, is used in the treatment of subjects suffering from hyperglycaemia.

## Patentansprüche

1. Pharmazeutische oder diätetische Zusammensetzung, geeignet zur oralen Verabreichung bei der Behandlung von fettleibigen oder übergewichtigen Personen, die an Hyperglykämie und/oder Hyperlipidämie leiden, die als Wirkstoff aktivierte Cellulose umfasst, welche durch heiße alkalische Behandlung von granulärer Cellulose, gefolgt von Abkühlung und pH-Neutralisierung, erhalten wird, und möglicherweise derivatisiert ist, um ein Konjugat zu bilden, welches ausgewählt ist aus der Gruppe bestehend aus Cellulose-Oleylamin-Glucosamin, allein oder in Kombination mit weiteren Wirkstoffen.

2. Zusammensetzung nach Anspruch 1, wobei die Behandlung der granulierten Cellulose bei einer Temperatur von ca. 90°C mit einer wässrigen Lösung einer geeigneten starken Base, vorzugsweise Natriumhydroxid, durchgeführt wird.

3. Zusammensetzung nach Anspruch 1, welche als Wirkstoff aktivierte Cellulose umfasst, die unter Bildung eines Konjugats derivatisiert ist, welches aus der Gruppe bestehend aus Cellulose-Oleylamin, Cellulose-Glucosamin und Cellulose-Oleylamin-Glucosamin ausgewählt ist.

4. Zusammensetzung nach Anspruch 1, welche aktivierte Cellulose in Kombination mit mindestens einem Konjugat davon, wie in Anspruch 1 definiert, umfasst.

5. Zusammensetzung nach einem der vorhergehenden Ansprüche, in granulierter Form, möglicherweise eingearbeitet in Lebensmittel, wie Kekse, Cracker, Brotstangen und dergleichen.

6. Die Zusammensetzung nach einem der vorhergehenden Ansprüche, welche ferner einen oder mehrere pharmazeutisch verträgliche Hilfsstoffe und/oder Exzipienten umfasst, welcher bzw. welche für die orale Verabreichung der Zusammensetzung geeignet ist bzw. sind.

7. Aktivierte Cellulose, erhältlich durch heiße alkalische Behandlung von granulierter Cellulose, gefolgt von Abkühlung und pH-Neutralisierung, möglicherweise derivatisiert, um ein Konjugat zu bilden, ausgewählt aus der Gruppe bestehend aus Cellulose-Oleylamin, Cellulose-Glucosamin und Cellulose-Oleylamin-Glucosamin, allein oder in Kombination mit weiteren Wirkstoffen, zur Verwendung bei der Behandlung von Fettleibigkeit oder Übergewicht bei fettleibigen oder übergewichtigen Probanden, und zur Verwendung bei der Behandlung von Hyperglykämie und/oder Hyperlipidämie bei Probanden, die an Hyperglykämie und/oder Hyperlipidämie leiden.

8. Aktivierte Cellulose zur Verwendung nach Anspruch 7, wobei die aktivierte Cellulose derivatisiert ist, um ein Konjugat zu bilden, das ausgewählt ist aus der Gruppe bestehend aus Cellulose-Oleylamin, Cellulose-Glucosamin und Cellulose-Oleylamin-Gluco-Samin.

9. Aktivierte Cellulose zur Verwendung nach den Ansprüchen 7 und 8, wobei die aktivierte Cellulose, ihr Derivat Cellulose-Oleylamin-Glucosamin, oder Kombinationen davon, bei der Behandlung von fettleibigen oder übergewichtigen Probanden verwendet wird bzw. werden.

10. Aktivierte Cellulose zur Verwendung nach den Ansprüchen 7 und 8, wobei das Derivat Cellulose-Oleylamin, allein oder in Kombination mit aktivierter Cellulose, bei der Behandlung von Personen verwendet wird, die an Hyperlipidämie leiden.

11. Aktivierte Cellulose zur Verwendung nach den Ansprüchen 7 und 8, wobei das Cellulose-Glucosamin-Derivat, allein oder in Kombination mit aktivierter Cellulose, bei der Behandlung von Personen verwendet wird, die an Hyperglykämie leiden.

## Revendications

1. Composition pharmaceutique ou alimentaire appropriée pour une administration orale dans le traitement de sujets obèses ou en surpoids souffrant d'hyperglycémie et/ou d'hyperlipidémie, comprenant en tant que principe actif une cellulose activée obtenue par traitement alcalin à chaud de cellulose granulaire, suivi d'un refroidissement et d'une neutralisation du pH, éventuellement dérivatisée pour former un conjugué choisi dans le groupe constitué de la cellulose-oléylamine-glucosamine, seule ou en combinaison avec d'autres principes actifs.

2. Composition selon la revendication 1 dans laquelle ledit traitement de la cellulose granulaire est réalisé à une température d'environ 90 °C avec une solution aqueuse d'une base forte appropriée, de préférence de l'hydroxyde de sodium.

3. Composition selon la revendication 1 comprenant en tant que principe actif une cellulose activée dérivatisée pour former un conjugué choisi dans le groupe constitué de la cellulose-oléylamine, de la cellulose-glucosamine et de la cellulose-oléylamine-glucosamine.

4. Composition selon la revendication 1 comprenant une cellulose activée en combinaison avec au moins un conjugué de celle-ci selon la revendication 1.

5. Composition selon l'une quelconque des revendications précédentes, sous forme granulaire, éventuellement incorporée dans un aliment, tel que des biscuits, des biscuits salés, des bâtonnets de pain et similaires.

6. Composition selon l'une quelconque des revendications précédentes comprenant en outre un ou plusieurs adjuvants et/ou excipients pharmaceutiquement acceptables appropriés pour une administration orale de la composition.

7. Cellulose activée obtenue par traitement alcalin à chaud de cellulose granulaire, suivi d'un refroidissement et d'une neutralisation du pH, éventuellement dérivatisée pour former un conjugué choisi dans le groupe constitué de la cellulose-oléylamine, de la cellulose-glucosamine et de la cellulose-oléylamine-glucosamine, seule ou en combinaison avec d'autres principes actifs, pour une utilisation dans le traitement de sujets obèses ou en surpoids, et pour une utilisation dans le traitement de l'hyperglycémie et/ou de l'hyperlipidémie chez des sujets souffrant d'hyperglycémie et/ou d'hyperlipidémie.

8. Cellulose activée pour une utilisation selon la revendication 7 dans laquelle ladite cellulose activée est dérivatisée pour former un conjugué choisi dans le groupe constitué de la cellulose-oléylamine, de la cellulose-glucosamine et de la cellulose-oléylamine-glucosamine.

9. Cellulose activée pour une utilisation selon les revendications 7 et 8 dans laquelle ladite cellulose activée, sa cellulose-oléylamine-glucosamine dérivée, ou des combinaisons de celles-ci, sont utilisées dans le traitement de sujets obèses ou en surpoids.

10. Cellulose activée pour une utilisation selon les revendications 7 et 8 dans laquelle ladite cellulose-oléylamine dérivée, seule ou en combinaison avec une cellulose activée, est utilisée dans le traitement de sujets souffrant d'hyperlipidémie.

11. Cellulose activée pour une utilisation selon les revendications 7 et 8 dans laquelle ladite cellulose-glucosamine dérivée, seule ou en combinaison avec une cellulose activée, est utilisée dans le traitement de sujets souffrant d'hyperlipidémie.
